# FASCICULE DE BREVET EUROPEEN

(11) **EP 1 438 922 B1**
(45) Date de publication et mention de la délivrance du brevet: **01.12.2010**
(21) Numéro de dépôt: 04290071.2
(22) Date de dépôt: 12.01.2004
(51) Int. Cl.: A61B 5/103, G06K 9/00, A61B 5/053

(54) **Dispositif et procédé visant à évaluer l'hydratation de la peau ou des muqueuses**
Vorrichtung und Verfahren zur Schätzung der Hydrierung von Haut und Schleimhaut
Device and method for evaluating the hydration of skin and mucosa

(30) Priorité: 14.01.2003 FR 0300367; 13.03.2003 FR 0303118
(43) Date de publication de la demande: 21.07.2004
(73) Titulaire: L'Oréal, 75008 Paris (FR)
(72) Inventeur: Leveque, Jean-Luc, 75017 Paris (FR); Querleux, Bernard, 94170 Le Perreux (FR); Giron, Franck, 77164 Ferrieres-en-Brie (FR)
(74) Mandataire: Tanty, François

(56) Documents cités:
- EP-A- 1 316 912
- WO-A-01/24700
- DE-A- 19 936 097
- FR-A- 2 821 541

## Description

La présente invention concerne les procédés et dispositifs visant à évaluer l'hydratation de la peau et/ou des muqueuses, notamment les lèvres.

Par « hydratation », on désigne la teneur en eau dans la peau et à sa surface, y compris l'eau provenant de la transpiration.

Le brevet US 6 370 426 décrit un dispositif permettant de mesurer l'hydratation de la peau humaine, comportant des moyens pour mesurer une variation d'impédance entre deux électrodes en contact électrique avec la peau.

La demande WO 02/056766 décrit également un dispositif assez similaire.

La société allemande COURAGE & KHAZAKA commercialise sous la référence CM825® un cornéomètre qui effectue une mesure de capacitance du stratum corneum. La mesure est fondée sur la différence de constante diélectrique qui existe entre l'eau et les autres substances présentes dans la peau, ce qui lie la capacitance à la proportion d'eau dans les couches superficielles de la peau.

Il a par ailleurs été proposé dans la demande EP 1 177 766 d'utiliser un capteur comportant un réseau de cellules de détection non optiques, notamment capacitives, pour obtenir une information concernant le micro-relief d'une région non dermatoglyphique du corps humain, par exemple la densité surfacique des lignes de la peau, le coefficient d'anisotropie de la densité de ces lignes ou le nombre et la taille des pores de la peau.

Les demandes WO 01/24700 et DE 199 36 097 décrivent des dispositifs et procédés d'authentification par analyse d'empreintes digitales.

La demande WO 01/24700 décrit l'analyse d'une variation éventuelle due à la transpiration de l'image acquise au moyen du capteur, afin de confirmer au système d'authentification que le doigt dont l'empreinte est analysée est bien vivant.

DE 199 36 097 décrit l'ajustement d'un seuil de sensibilité de la reconnaissance d'empreintes digitales en fonction de l'humidité de la peau.

Ces documents concernent des dispositifs et procédés qui ne visent pas à délivrer une information concernant l'hydratation de la peau sous une forme directement appréhendable par un individu, afin que celui-ci soit renseigné sur l'état de sa peau.

La présente invention vise à proposer un nouveau dispositif permettant de délivrer au moins une information relative à l'hydratation d'une région de la peau ou des muqueuses.

Ce dispositif comporte un capteur comportant un réseau de cellules de détection non optiques, notamment capacitives, et un dispositif de traitement agencé pour délivrer l'information ci-dessus à partir de signaux provenant du capteur. Cette information est délivrée par le dispositif sous une forme directement appréhendable par un individu, par exemple un message visuel et/ou sonore, par exemple imprimé ou affiché sur un écran. Un tel message peut comporter entre autres une note ou un qualificatif tel que « peau suffisamment hydratée », « peau insuffisamment hydratée » ou d'autres termes encore.

Les signaux provenant du capteur peuvent être analogiques ou numériques. Le capteur peut notamment délivrer une valeur codée sur un certain nombre de bits, par exemple 8 bits, associée à chaque cellule de détection.

La demanderesse a constaté que les signaux délivrés par le capteur pouvaient être représentatifs de l'hydratation de la région examinée.

Le capteur utilisé peut avantageusement présenter une résolution spatiale meilleure que 100 µm, notamment égale à ou meilleure que 50 µm, dans au moins une direction, et de préférence dans deux directions orthogonales d'un plan.

Le capteur peut comporter par exemple plus de 200 x 200 cellules de détection et le réseau de cellules de détection peut occuper une surface comprise par exemple entre 10 x 10mm² et 20 x 20mm².

Le réseau de cellules du capteur est avantageusement bidimensionnel, chaque cellule étant adressable individuellement.

Le dispositif selon l'invention peut être agencé, dans une réalisation particulière, pour délivrer au moins une image de la région analysée par le capteur, l'intensité de gris de chaque pixel de l'image étant représentative de la capacitance mesurée par une cellule de détection. Un niveau de gris O correspond par exemple à du noir sur l'image et un niveau de gris maximal, par exemple égal à 255 dans le cas d'un codage sur 8 bits, à du blanc, les niveaux de gris entre ces valeurs extrêmes correspondant respectivement aux diverses nuances intermédiaires.

Le dispositif peut être agencé pour délivrer et analyser une succession d'images d'une même région de peau ou de muqueuses, afin par exemple d'évaluer la transpiration, comme cela sera détaillé dans la suite.

Le dispositif de traitement peut être agencé pour effectuer un traitement statistique de l'image et ladite information peut résulter au moins de ce traitement statistique. L'information qui est délivrée peut, le cas échéant, l'être après recoupement des résultats tirés du traitement de l'image avec d'autres données concernant par exemple l'âge, le sexe ou le type ethnique de la personne concernée.

Le traitement statistique peut comporter une analyse des niveaux de gris de l'image et notamment la détermination du niveau de gris partagé par le plus grand nombre de pixels de l'image. Le traitement statistique peut encore, par exemple, faire intervenir le calcul d'un niveau de gris moyen de l'image ou d'au moins une portion de celle-ci.

Dans une variante de mise en oeuvre de l'invention, le traitement statistique comporte l'établissement d'une matrice de co-occurrence au moins.

Cette matrice peut être déterminée sur la base d'une image traitée par érosion et ré-échantillonnage.

L'homogénéité de l'hydratation peut être déterminée à partir d'au moins la connaissance de la matrice de co-occurrence ; l'aire de la matrice de co-occurrence peut notamment être considérée comme étant représentative de l'homogénéité de l'hydratation.

L'utilisation d'une matrice de co-occurrence permet, d'une certaine manière, de tenir compte du positionnement relatif des zones foncées par rapport aux zones claires de l'image.

Le traitement statistique peut encore comporter une décomposition de l'image en sous-ensembles ayant une taille donnée, le calcul d'un niveau de gris moyen pour chaque sous-ensemble et le calcul de la variance de ces niveaux de gris moyen.

On peut calculer la variance pour différentes tailles de sous-ensembles et déterminer la taille pour laquelle la variance est maximum.

Cette taille peut être représentative de la taille des inhomogénéités de l'image.

L'information délivrée peut comporter une indication relative à l'homogénéité de l'hydratation.

L'inhomogénéité de l'hydratation peut traduire par exemple l'existence de gouttelettes de sueur à la surface de la peau. L'inhomogénéité de l'hydratation peut encore être liée, par exemple, à l'existence de taches dues au photo-vieillissement.

Pour évaluer l'inhomogénéité de l'hydratation, on peut par exemple déterminer les niveaux de gris moyens de sous-ensembles, de préférence non sécants, de l'image et calculer une valeur représentative d'un écart entre ces niveaux de gris moyens, par exemple l'écart-type.

Les différents sous-ensembles peuvent être répartis de diverses manières au sein de l'image, étant par exemple formés par un quadrillage d'au moins une partie de l'image. Les sous-ensembles peuvent encore être disposés sur l'image d'une façon non régulière, en des emplacements sélectionnés sur l'image par exemple en raison d'une valeur particulière, en ces emplacements, des niveaux de gris, ces derniers étant par exemple compris dans une plage donnée. Les sous-ensembles peuvent par exemple être localisés sur l'image de façon à ce que les niveaux de gris moyens correspondants comportent des valeurs extrêmes présentant des écarts significatifs.

Le dispositif de traitement peut être agencé pour comparer l'image avec une banque d'images et, au moins à partir de cette comparaison, en tirer une information utile pour évaluer l'état de la peau ou des muqueuses. L'accès à cette banque d'images peut s'effectuer le cas échéant à travers un réseau informatique ou de téléphonie.

Le dispositif de traitement peut être agencé pour que le capteur analyse ladite région pendant une durée prédéfinie, qui peut être brève. Cette durée peut être comprise entre 1 et 30 secondes, par exemple, notamment entre 2 et 10 secondes, voire entre 3 et 7 secondes. Lorsque l'on souhaite observer l'évolution de l'hydratation dans le temps, la durée peut être plus longue que lorsque l'on désire seulement mesurer l'hydratation à un instant donné.

Le dispositif de traitement peut être agencé pour délivrer une indication relative à l'état de vieillissement de la peau.

Le dispositif de traitement peut notamment être agencé pour permettre de déterminer deux orientations prépondérantes des lignes de la peau relativement à l'axe du bras par exemple et l'écart existant entre ces orientations, et délivrer à partir au moins dudit écart une indication relative à l'état de vieillissement de la peau, la demanderesse ayant constaté que cet écart est généralement fonction de l'âge des individus, diminuant avec l'âge.

Par « lignes de la peau », on désigne les lignes formées par les sillons s'étendant entre les plateaux de la peau.

Le dispositif peut être agencé pour échanger des données par une liaison filaire ou non avec un ordinateur personnel ou un terminal portable, notamment un téléphone portable ou un assistant personnel numérique.

Le dispositif peut, le cas échéant, comporter au moins un second capteur d'un type différent, notamment un capteur de conductivité, de température, de couleur, d'élasticité, de pH ou un biocapteur.

L'invention a encore pour objet, selon un autre de ses aspects, un procédé pour évaluer l'hydratation d'une région de la peau ou des muqueuses, comportant les étapes suivantes :
- appliquer sur ladite région un capteur comportant un réseau de cellules de détection non optiques, notamment capacitives,
- recueillir des signaux provenant dudit capteur et délivrer à partir de ces signaux une information relative à l'hydratation de ladite région, ainsi éventuellement qu'à son vieillissement.

Ce procédé peut comporter l'édition d'un message contenant ladite information, un tel message étant par exemple visuel et/ou sonore, notamment imprimé ou affiché sur un écran.

Le message peut comporter des chiffres, des lettres, des mots ou tout autre élément intelligible.

Préalablement à l'application du capteur sur la peau, celle-ci peut être nettoyée et/ou séchée au moyen d'un buvard, par exemple, afin d'éviter la présence initiale de gouttelettes de sueur.

On peut en outre déterminer, à partir des signaux délivrés par le capteur, l'inhomogénéité de l'hydratation et notamment évaluer l'importance de la transpiration en fonction de cette inhomogénéité, la transpiration se traduisant par une hydratation inhomogène, liée à l'apparition localement de gouttelettes de sueur. L'étude de l'homogénéité de l'hydratation peut être également utile le cas échéant pour délivrer une information relative au photovieillissement cutané, par exemple.

On peut déterminer l'information relative à l'hydratation ou au vieillissement de ladite région à partir au moins d'un traitement statistique d'au moins une image obtenue à l'aide dudit capteur. Cette image peut être affichée sur un écran ou imprimée, mais l'image peut encore ne pas être visualisée et être, le cas échéant, enregistrée sur un support d'information tel que par exemple un disque dur ou un disque optique.

Comme évoqué plus haut, on peut analyser ladite région avec le capteur pendant une durée prédéfinie, qui peut être plus ou moins longue selon que l'on souhaite évaluer l'hydratation à un instant donné ou suivre l'évolution de celle-ci dans le temps, afin par exemple de quantifier la transpiration.

On peut délivrer en outre, à partir des signaux provenant du capteur, une indication relative à la densité des pores de la peau et/ou à leur taille.

On peut aussi déterminer à partir des signaux délivrés par le capteur deux orientations prépondérantes des lignes de la peau, et calculer un écart entre lesdites orientations. La connaissance d'un tel écart peut être utile pour évaluer le vieillissement de la peau.

On peut comparer les résultats de deux évaluations relatives à l'hydratation de ladite région à deux instants différents et délivrer une indication liée à l'évolution de l'hydratation entre ces deux instants. Cela peut permettre, par exemple, de renseigner l'individu objet des évaluations sur l'incidence d'un traitement.

On peut par exemple évaluer l'hydratation d'une région de la peau ou des muqueuses à un premier instant, effectuer un traitement visant à hydrater cette région, par exemple appliquer une crème hydratante, puis effectuer une deuxième évaluation à un instant ultérieur, par exemple quelques heures ou jours après, afin de déterminer l'incidence du traitement.

On peut encore, par exemple, évaluer la transpiration à un premier instant, effectuer un traitement visant à réduire la transpiration, par exemple appliquer un déodorant, puis effectuer une deuxième évaluation à un instant ultérieur, et déduire de la comparaison des évaluations une information relative à l'efficacité du traitement.

On peut aussi effectuer au moins une évaluation de l'hydratation d'une région du corps non exposée à un environnement donné, par exemple non exposée au soleil, et au moins une évaluation d'une région du corps exposée à cet environnement et comparer les résultats, et déterminer à partir de la comparaison de ces résultats une information utile pour évaluer l'incidence de cet environnement sur ladite région et le cas échéant le vieillissement de la peau, par exemple.

Par ailleurs, on peut analyser ladite région avec le capteur en un premier emplacement géographique, par exemple dans un institut de beauté, sur un point de vente ou à domicile, et transmettre à distance, par un réseau Internet, Intranet ou de téléphonie mobile, des données obtenues avec le capteur, et traiter à un deuxième emplacement géographique, par exemple dans un centre de recherche, lesdites données, de manière à évaluer l'hydratation de ladite région.

On peut transmettre le résultat de l'évaluation par un réseau Internet, Intranet ou de téléphonie mobile. On peut également transmettre le résultat de l'évaluation par courrier. Le résultat de l'évaluation peut être accompagné, le cas échéant, par la prescription d'un produit ayant une action sur l'hydratation de ladite région, par exemple un produit antiperspirant ou hydratant.

Pour effectuer les acquisitions, le capteur peut être appliqué sur une région non dermatoglyphique du corps, notamment sur l'avant-bras.

Dans un exemple de mise oeuvre de l'invention, on mémorise des images successives dans le temps de la région étudiée, obtenues à partir dudit capteur, et/ou des informations relatives à l'état de l'hydratation et/ou à l'homogénéité de l'hydratation. La mémorisation des images peut être effectuée par exemple dans le but de les comparer.

On peut ainsi comparer, en différé par exemple, au moins deux valeurs obtenues à partir d'un traitement de deux images successives, afin de mettre en évidence une amélioration ou une dégradation dans le temps de l'état de la peau, notamment de son hydratation, ou encore évaluer la transpiration.

L'invention a encore pour objet, selon un autre de ses aspects, un procédé de prescription d'un produit, notamment un produit cosmétique, pouvant comporter les étapes suivantes :
- évaluer l'hydratation d'une région de la peau ou des muqueuses en mettant en oeuvre le procédé tel que défini plus haut,
- prescrire au vu du résultat de l'évaluation un produit ayant un effet sur l'hydratation de ladite région.

Par « produit cosmétique », on désigne un produit tel que défini dans la Directive 93/35/CEE du Conseil du 14 juin 1993. Un lait et une crème pour le corps ou le visage, visant à hydrater la peau, sont des exemples de produits cosmétiques, de même qu'un antiperspirant.

L'invention a encore pour objet, selon un autre de ses aspects, un procédé pour déterminer l'efficacité d'un traitement ayant une action sur l'hydratation de la peau, comportant les étapes suivantes :
- effectuer une première évaluation de l'hydratation de la peau,
- effectuer le traitement,
- effectuer après le traitement, une deuxième évaluation de l'hydratation de ladite région,
- l'une au moins des première et deuxième évaluations étant effectuée en mettant en oeuvre le procédé tel que défini plus haut. De préférence, les deux évaluations sont effectuées en mettant en oeuvre le même procédé.

L'invention a encore pour objet, selon un autre de ses aspects, un procédé de traitement d'une région du corps, comportant les étapes suivantes :
- évaluer l'hydratation de ladite région en mettant en oeuvre le procédé tel que défini plus haut,
- effectuer un traitement ayant une action sur l'hydratation de ladite région au vu du résultat de l'évaluation. Ce traitement peut s'effectuer par voie topique, orale ou autre. Le traitement peut aussi comporter le suivi d'un régime alimentaire ou sportif particulier ou l'administration de soins spécifiques tels que des massages.

L'invention a encore pour objet, selon un autre de ses aspects, une méthode pour promouvoir la vente d'un produit, notamment cosmétique, comportant l'étape consistant à faire état d'une activité ou d'une efficacité du produit mise en évidence par un dispositif ou par un procédé tel que défini plus haut.

Une telle promotion du produit pourra se faire par n'importe quel canal de communication. Elle pourra être faite notamment par un vendeur, directement sur le point de vente, par la radio, la télévision ou le téléphone, notamment dans le cadre de spots publicitaires ou de messages courts. Elle pourra être faite également par le canal de la presse écrite ou par le biais de tout autre document, en particulier à des fins publicitaires. Elle pourra se faire également par Internet, par tout autre réseau informatique adéquat ou par un réseau de téléphonie mobile. Elle pourra être faite également directement sur le produit, notamment sur son packaging ou sur toute notice explicative qui lui est associée.

L'invention a encore pour objet l'utilisation d'un capteur d'empreintes digitales à réseau de cellules de détection capacitives, intégré dans un micro-ordinateur ou un terminal portable, pour évaluer l'hydratation de la peau.

L'invention a ainsi pour objet, selon l'un de ses aspects, un téléphone portable équipé d'un capteur comportant un réseau de cellules de détection capacitives destiné à être appliqué sur une région de la peau ou des muqueuses, ce téléphone étant agencé pour traiter des signaux délivrés par ledit capteur afin de délivrer une information relative à l'hydratation de la peau ou des muqueuses.

Cette information peut être délivrée par exemple sous la forme d'un message visuel et/ou sonore ou par l'envoi d'un fichier de type « Short Message Service » ou « Multimedia Messaging Service » à un centre de traitement de l'information, qui en retour délivre un message visuel et/ou sonore, notamment imprimé ou affiché sur un écran.

L'invention pourra être mieux comprise à la lecture de la description détaillée qui va suivre, d'exemples de mise en oeuvre non limitatifs de celle-ci, et à l'examen du dessin annexé, sur lequel :
- la figure 1 représente de manière schématique un exemple de dispositif selon l'invention,
- la figure 2 représente une variante du dispositif se présentant sous la forme d'un appareil portatif,
- la figure 3 représente de manière schématique un exemple de téléphone portable équipé d'un capteur à réseau de cellules de détection non optiques,
- la figure 4 représente de manière partielle et schématique en vue de face un exemple de capteur,
- la figure 5 représente de manière schématique et partielle en coupe deux cellules de détection du capteur,
- la figure 6 représente un exemple d'image pouvant être obtenue avec le capteur de la figure 4,
- la figure 7 représente un exemple d'histogramme des niveaux de gris,
- la figure 8 illustre une possibilité de délimitation de zones élémentaires en vue d'évaluer l'inhomogénéité de l'hydratation,
- la figure 9 représente un autre exemple de délimitation de zones élémentaires,
- la figure 10 illustre de façon schématique et partielle une possibilité d'évolution d'un histogramme au cours du temps,
- la figure 11 illustre le positionnement du capteur sur un avant-bras,
- la figure 12 représente un exemple de distribution de l'orientation des lignes de la peau relativement à l'axe longitudinal du bras,
- la figure 13 représente une image après érosion,
- la figure 14 est un histogramme des niveaux de gris de l'image de la figure 13,
- la figure 15 représente la matrice de co-occurrence obtenue à partir de l'image de la figure 13, après ré-échantillonnage, et
- les figures 16 à 18 sont des vues respectivement analogues aux figures 13 à 15, obtenues à partir d'une autre image de départ.

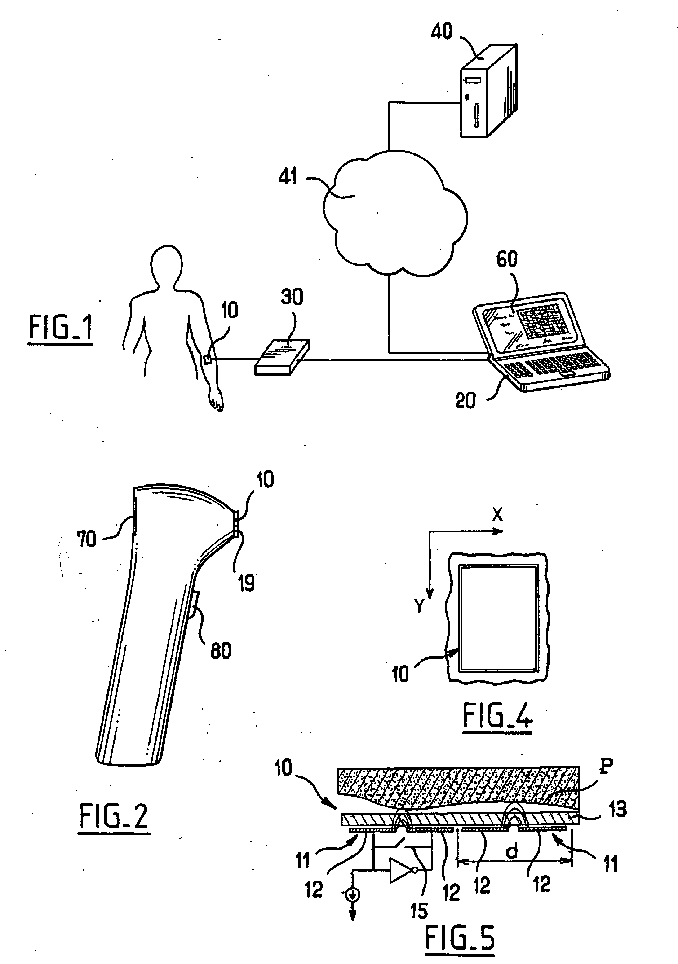

On a représenté à la figure 1 un exemple de dispositif selon l'invention, comportant un capteur 10 destiné à analyser une région de peau ou de muqueuses et des moyens de traitement des signaux délivrés par le capteur 10, ces moyens de traitement comportant par exemple un micro-ordinateur 20 conventionnel et éventuellement une interface 30 permettant de transmettre des données issues du capteur 10 au micro-ordinateur 20. Ce dernier peut par ailleurs être relié, le cas échéant, à un serveur distant 40 par un réseau informatique 41 Internet ou Intranet.

Dans l'exemple illustré, l'interface 30 a été représentée sous la forme d'un appareil distinct du capteur 10 et du micro-ordinateur 20 mais on ne sort pas du cadre de la présente invention lorsque l'interface 30 est intégrée dans le capteur 10 ou dans le micro-ordinateur 20.

Bien entendu, le micro-ordinateur 20 n'est qu'un exemple parmi d'autres de moyens de traitement pouvant être utilisés et dans la variante illustrée à la figure 2 le dispositif permettant d'évaluer l'hydratation se présente sous la forme d'un appareil portable, tenant dans une main, pouvant comporter outre le capteur 10 un écran ou afficheur 70 ainsi par exemple qu'un bouton-poussoir 80 permettant de déclencher un cycle d'acquisition des données.

Le cas échéant, on peut associer au capteur 10 que ce soit dans la réalisation de la figure 1 ou celle de la figure 2 au moins un capteur annexe 19, par exemple de pH, de température, d'élasticité ou de couleur, et combiner éventuellement l'information relative à l'hydratation provenant du capteur 10 avec d'autres informations provenant de ce ou ces capteur(s) annexe(s) afin d'obtenir une meilleure appréciation de l'état de la peau.

Le micro-ordinateur 20 de l'exemple de la figure 1 pourrait encore être remplacé par un terminal portable, par exemple un téléphone mobile ou un assistant personnel numérique.

Le capteur 10 peut éventuellement être intégré au micro-ordinateur 20, au téléphone ou à l'assistant personnel afin de servir également à identifier l'utilisateur.

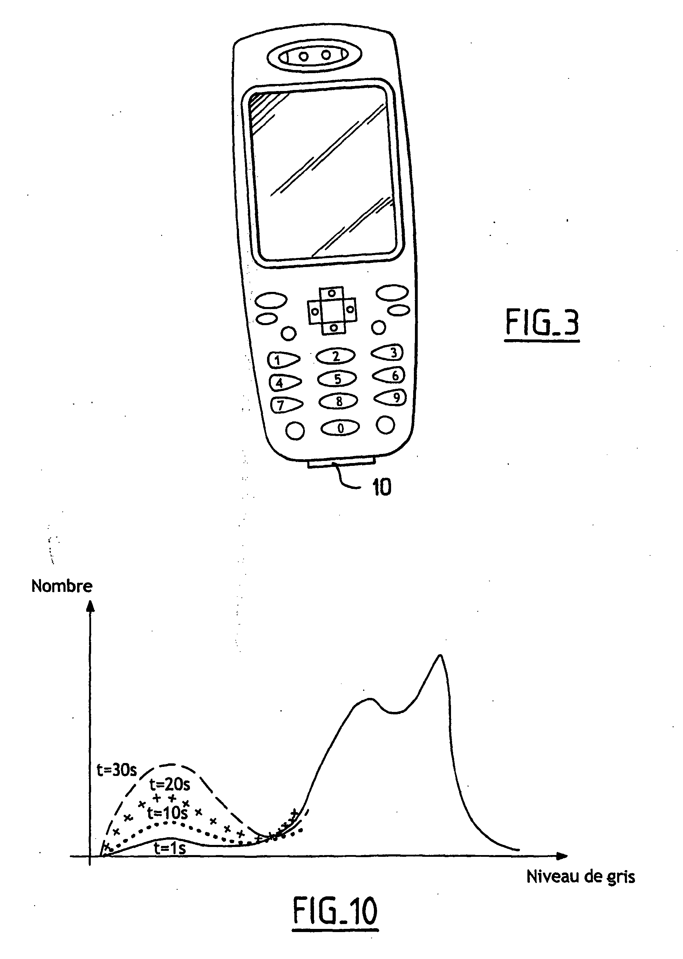

A titre d'illustration, on a représenté à la figure 3 un téléphone portable dans lequel est intégré le capteur 10, par exemple à une extrémité ou sur un côté du boîtier afin de faciliter l'application du capteur 10 sur l'avant-bras, par exemple.

Dans une variante encore, l'interface 30 est agencée pour se raccorder au réseau informatique 41 et transmettre directement les données provenant du capteur 10 au serveur 40, lequel est agencé pour effectuer le traitement de ces données.

Le capteur 10, qui a été représenté plus particulièrement sur les figures 4 et 5, comporte un réseau bidimensionnel, s'étendant dans un plan X Y, de cellules de détection capacitives 11.

Ce capteur 10 est par exemple un capteur commercialisé sous la marque TOUCHCHIP® par la société ST-MICROELECTRONICS.

Chaque cellule de détection 11 comporte deux plaques métalliques adjacentes 12 qui sont séparées de la région analysée, par exemple la peau P, par un revêtement protecteur 13 en un isolant électrique. Des lignes de champ électrique s'étendent à travers le revêtement 13 entre les plaques 12 et lorsque la peau P est au contact du revêtement 13 et se situe à proximité des plaques 12, le micro-relief de la peau P interfère avec les lignes de champ et modifie la capacité du condensateur formé par les deux plaques 12.

Dans le cas du capteur TOUCHCHIP® ci-dessus, les cellules de détection 11 opèrent en deux phases. Dans une phase initiale, les deux plaques 12 sont reliées par un interrupteur électronique 15 ce qui permet d'effectuer une initialisation, puis dans une phase de détection, l'interrupteur 15 est ouvert et une capacitance est détectée, laquelle dépend notamment du micro-relief de la peau P et de la constante diélectrique de cette dernière.

Les signaux délivrés par le capteur 10 peuvent se présenter sous la forme de données numériques, le capteur 10 pouvant intégrer un convertisseur A/D.

Une valeur représentative de la capacitance mesurée par une cellule de détection 11, encore appelée pixel, peut être lue à une adresse particulière, par un adressage de type RAM.

La dimension d d'une cellule de détection 11 peut être inférieure ou égale à 50 µm, et la résolution de l'image meilleure que 500 dpi.

Dans l'exemple considéré, le capteur 10 comporte un réseau de 256 x 360 cellules, pour une surface active ayant des dimensions de 18 x 12,8 mm.

Le capteur 10 permet d'obtenir une image 60 de la région analysée, laquelle peut être affichée le cas échéant, comme illustré sur la figure 1, sur l'écran du micro-ordinateur 20 ou sur celui du téléphone portable de la figure 3. L'image affichée peut être agrandie, par exemple d'un facteur 2 ou plus, notamment 6. On ne sort pas du cadre de la présente invention lorsqu'aucune image n'est affichée.

L'information délivrée par le capteur 10, relative à chaque pixel de l'image, est par exemple une valeur numérique codée sur 8 bits, représentative de l'amplitude de la variation de capacitance détectée localement.

L'image 60 comprend ainsi des niveaux de gris compris entre 0 et 255 dans l'exemple considéré.

La demanderesse a pu mettre en évidence que ces niveaux de gris étaient représentatifs de l'état d'hydratation de la région de peau ou de muqueuses analysée par le capteur 10, de sorte que ce dernier peut permettre d'établir une cartographie de l'hydratation du stratum corneum.

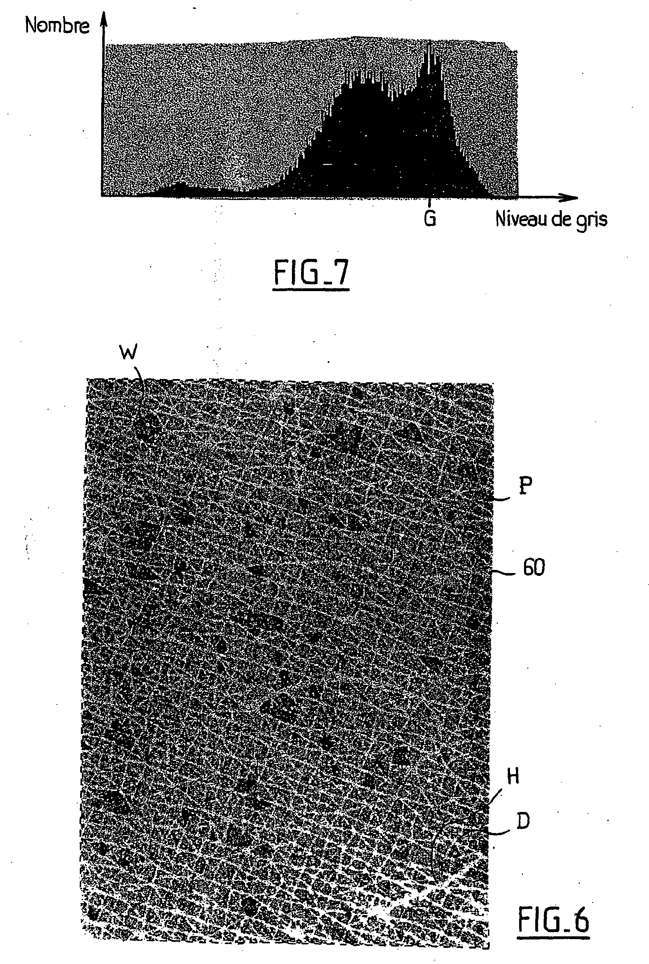

La figure 6 représente un exemple d'image 60.

On peut discerner sur cette figure les lignes D de peau, les pores T, des poils H, ainsi que des taches W dues à la présence de gouttelettes de sueur.

La figure 7 représente un histogramme des niveaux de gris de cette image en fonction du nombre de pixels.

Une corrélation a pu être mise en évidence notamment entre le niveau de gris G partagé par le plus grand nombre de pixels et l'hydratation mesurée avec un cornéomètre conventionnel, par exemple CM 825® , ce qui peut permettre un étalonnage, le cas échéant.

Plutôt que de déterminer quel est le niveau de gris partagé par le plus grand nombre de pixels, on pourrait encore calculer le niveau de gris moyen des pixels de l'image, par exemple.

Le dispositif peut être agencé de telle sorte que la durée pendant laquelle le capteur 10 analyse la région de peau soit prédéfinie, étant par exemple égale à 5 secondes.

La durée peut être plus longue, comme cela sera détaillé dans la suite, afin par exemple de quantifier la transpiration.

L'image de capacitance délivrée par le capteur 10 permet également de déterminer le degré d'homogénéité de l'hydratation.

Dans une première approche, on peut considérer que l'écart-type entre les valeurs de gris de portions spécifiques d'une image obtenue au moyen du capteur 10 est représentatif de l'homogénéité de l'hydratation.

Plus précisément, pour évaluer l'inhomogénéité, on peut par exemple décomposer l'image ou une portion de celle-ci en sous-ensembles 100 comportant chacun par exemple entre 500 et 1 500 pixels, par exemple 1 024 pixels dans le cas de carrés de 32 pixels de côtés.

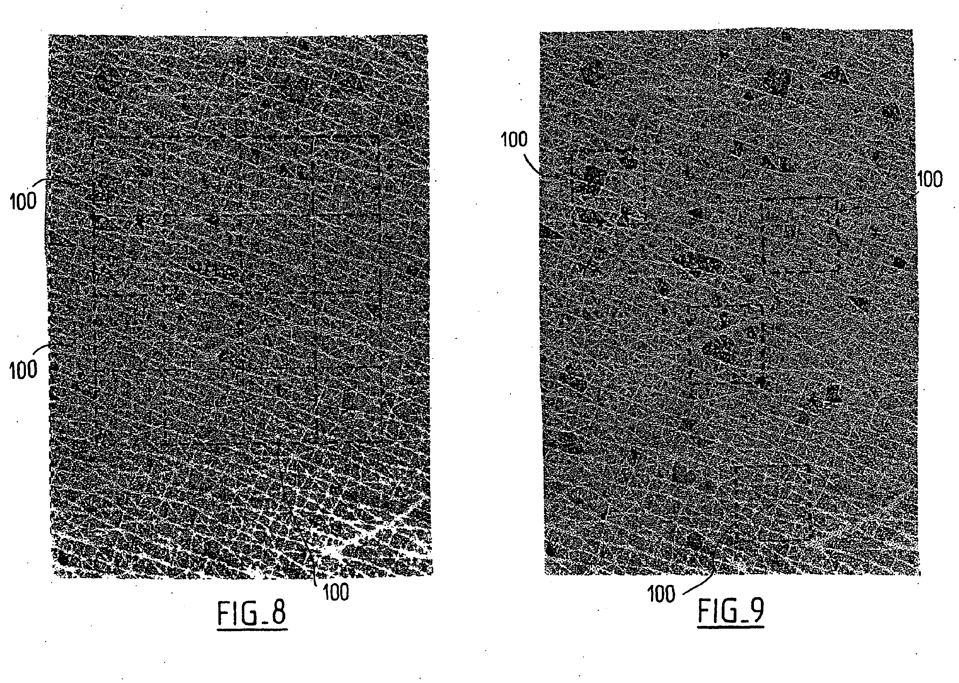

Ces sous-ensembles 100 peuvent être disposés sur l'image 60 de manière régulière comme illustré sur la figure 8 ou autrement, comme illustré sur la figure 9.

Sur la figure 8, les sous-ensembles 100 sont délimités par un quadrillage de l'image 60.

Pour chaque sous-ensemble 100, on calcule le niveau de gris moyen des pixels constituant ce sous-ensemble.

On obtient un ensemble de valeurs pour lesquelles on peut calculer l'écart-type. Cet écart-type est représentatif de l'inhomogénéité de l'image.

On peut déterminer l'inhomogénéité de l'image pour différentes tailles de sous-ensembles 100, par exemple au moins quatre tailles différentes.

On peut ainsi déterminer l'inhomogénéité pour des sous-ensembles 100 comportant par exemple 30 x 30 pixels, puis pour des sous-ensembles 100 comportant 20 x 20 pixels, 10 x 10 pixels et 5 x 5 pixels.

On obtient un écart-type maximum pour une taille donnée de sous-ensemble 100 et la taille en question peut fournir une information supplémentaire sur l'homogénéité de l'hydratation, l'écart-type maximum étant représentatif de la taille moyenne des inhomogénéités d'hydratation.

Sur la figure 9, l'emplacement des sous-ensembles 100 est déterminé de façon à obtenir une grande disparité entre les valeurs moyennes de niveaux de gris correspondant à ces sous-ensembles 100. Ces derniers peuvent être localisés par exemple surtout dans les zones les plus claires et les plus sombres de l'image. On peut par exemple ne positionner des sous-ensembles 100 que d'une part dans des zones de l'image comportant au moins un certain nombre de taches W liées à la transpiration, par exemple au moins deux, et d'autre part dans d'autres zones n'en comportant aucune.

Selon une autre approche, on peut procéder comme suit pour évaluer l'homogénéité de l'hydratation.

On peut commencer, le cas échéant, par délimiter à l'intérieur de l'image acquise par le capteur une zone excluant les bords de l'image afin de tenir compte de l'inhomogénéité d'application du capteur sur la peau dans la région des bords.

Ensuite, l'image peut être traitée de manière à éliminer les lignes de la peau par érosion. On peut utiliser par exemple la fonction *gray morphology erode* de la plateforme de traitement d'images IMAQ VISION BUILDER de National Instruments. Lors du traitement d'érosion, les pixels de l'image sont transformés en pavés. On peut choisir une matrice d'érosion de forme carrée comportant 7 x 7 pixels.

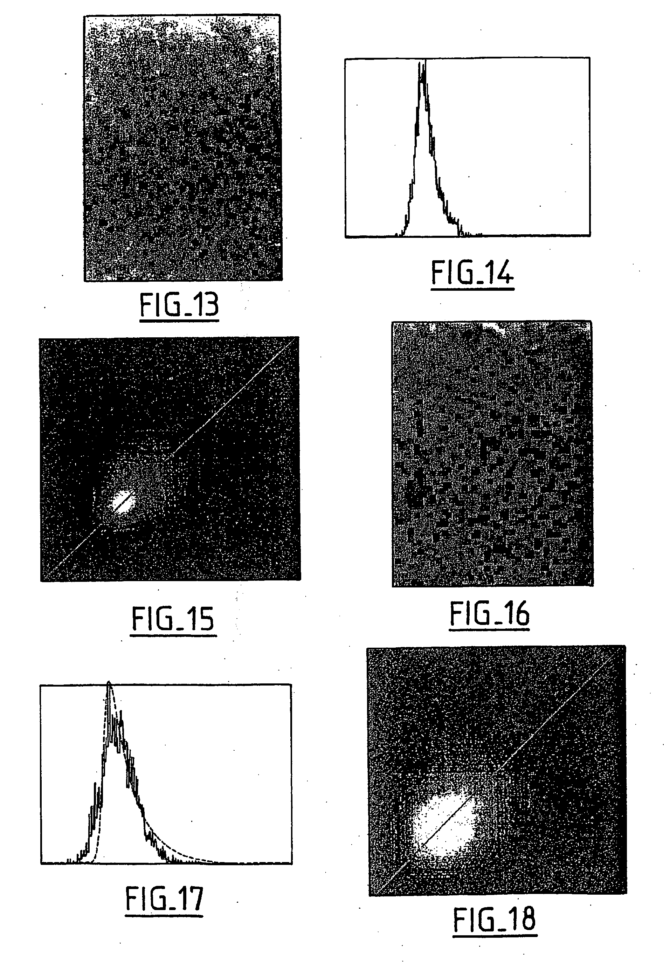

La figure 13 et la figure 16 sont des images obtenues après un tel traitement d'érosion. L'avantage de procéder au traitement d'érosion est d'éviter de prendre en considération, pour apprécier l'homogénéité de l'hydratation, les zones de la peau qui ne sont pas en contact avec le capteur.

On peut ensuite ne garder qu'un pixel par matrice, c'est-à-dire procéder à un ré-échantillonnage.

On obtient ainsi une image comportant un nombre relativement faible de pixels.

Cette image peut être traitée pour faire apparaître l'histogramme des niveaux gris, c'est-à-dire en abscisses les niveaux de gris allant par exemple de 0 à 255 et en ordonnées le nombre de pixels ayant un niveau de gris correspondant à une abscisse donnée.

On peut également construire à partir d'une telle image une matrice de co-occurrence.

Pour construire une matrice de co-occurrence, on extrait de l'image des pavés de pixels distants d'une certaine distance sur l'image dans une direction donnée. Cette direction peut par exemple être une verticale sur l'image et la distance correspondre à 5 pixels. Pour une paire de pixels ayant des niveaux de gris respectifs i et j, on trace un point à l'abscisse i et à l'ordonnée j. On procède ainsi pour tous les pixels de l'image.

La matrice obtenue est symétrique par rapport à la droite X = Y.

On a représenté sur les figures 15 et 18 des matrices de co-occurrence correspondant à différentes images de départ.

L'aire de la matrice est d'autant plus importante que l'image est inhomogène Ainsi, pour les matrices des figures 15 et 18 on a respectivement des aires de 2030 et 4640.

Un avantage du traitement par matrice de co-occurrence est de pouvoir choisir l'échelle d'analyse des variations en ajustant la distance inter pixel. Il est possible par exemple de diminuer l'incidence, sur le résultat final, des variations à très faible échelle des valeurs de niveaux de gris, variations qui ne sont pas forcément porteuses d'une information sur l'homogénéité de l'hydratation.

L'image de capacitance peut encore permettre de mettre en évidence des taches qui correspondent à des zones plus ou moins kératosiques, dont l'origine peut être liée à une photo-exposition.

Le dispositif selon l'invention peut être agencé pour permettre d'acquérir une séquence d'images à des intervalles de temps prédéfinis et enregistrer, pour chaque image de la séquence, l'histogramme par exemple, voire l'image complète.

On a illustré à la figure 10 un exemple d'évolution pour les faibles niveaux de gris d'un histogramme au cours du temps, lorsque le capteur 10 est laissé sur la peau.

On peut remarquer que le nombre de pixels correspondant aux faibles niveaux de gris tend à augmenter au cours du temps, ce qui peut s'expliquer par la transpiration qui continue pendant l'observation et qui tend à faire grossir les taches W sur l'image et/ou à augmenter leur nombre.

La transpiration peut être facilitée par la présence du capteur 10 qui présente un caractère occlusif.

La comparaison de deux séquences d'histogrammes avant et après un traitement visant à diminuer la transpiration peut par exemple permettre de vérifier l'efficacité de ce traitement.

Une séquence d'histogrammes peut être traitée par exemple afin de déterminer le taux de croissance des pixels dans les faibles niveaux de gris.

En fonction de ce taux, il est possible par exemple de déterminer si le sujet a tendance à transpirer ou non et lui recommander un produit ou un traitement, le cas échéant.

En comparant les taux mesurés avant et après traitement, on peut déterminer l'incidence du traitement sur la transpiration.

Les données délivrées par le capteur 10 peuvent être traitées de manière à fournir des informations autres que purement relatives à l'hydratation de la région analysée, par exemple des informations concernant le micro-relief de la peau, notamment le nombre et la taille des pores de la peau ainsi que la densité surfacique des lignes de la peau ou le coefficient d'anisotropie de la densité des lignes de la peau.

On pourra utilement se référer à ce sujet à la demande de brevet européen EP 1 177 766 de la demanderesse, dont le contenu est incorporé par référence.

Le coefficient d'anisotropie de la densité correspond au rapport de la densité des lignes D entourant les plateaux de la peau dans une première direction à la densité dans une deuxième direction, perpendiculaire à la première. Ce rapport évolue avec l'âge et permet de tirer une information sur l'état de vieillissement de la peau, par exemple.

On peut encore traiter l'image de manière à déterminer deux directions selon lesquelles sont orientées de façon prépondérante les lignes de la peau. On suppose ici que l'image est obtenue à l'aide d'un capteur comportant un réseau de cellules de détection capacitives mais d'autres types de capteurs à cellules de détection non optiques, par exemple thermiques ou à mesure de conductivité, pourraient être utilisés.

Pour déterminer les orientations prépondérantes des lignes de la peau, on peut procéder par exemple de la façon suivante.

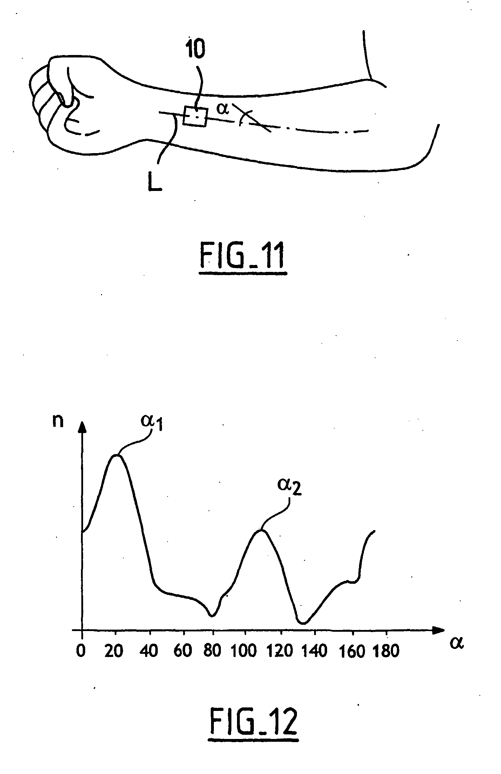

On peut commencer par appliquer le capteur 10 sur la face ventrale de l'avant-bras par exemple, avec le grand côté du capteur 10 parallèle à l'axe longitudinal L du bras, comme illustré à la figure 11.

On peut ensuite acquérir une image, dont la taille en pixels est par exemple 256 x 360. Sur cette image, on définit une région d'intérêt, circulaire, dont le diamètre est par exemple de 248 pixels.

On effectue ensuite une première étape de pré-traitement pour annuler notamment les inhomogénéités spatiales des bas niveaux de signal, dues à un contact non homogène sur toute la surface d'acquisition.

On réduit ensuite le nombre de niveaux de gris de 256 à 5 par exemple en utilisant un algorithme de nuées dynamiques, qui consiste à répartir les niveaux de gris en 5 niveaux les plus informatifs de l'image.

Sur l'image à 5 niveaux, on utilise une méthode dite de co-occurrence qui consiste à rechercher dans une direction d'étude fixée un motif moyen dans l'image affichée. On renouvelle la méthode en faisant varier l'angle α que fait la direction d'étude avec l'axe longitudinal L.

Un exemple de résultat est reproduit à la figure 12, sous la forme d'une courbe avec en ordonnées le nombre n de lignes de l'image pour une orientation donnée α en abscisses.

Deux maxima d'abscisses respectives α₁ et α₂ sont clairement visibles, correspondant respectivement à des valeurs de 20° et 110° environ.

L'écart α₂ ― α₁ entre les maxima évolue en fonction de l'état de vieillissement de la peau, tendant à diminuer avec l'âge. Ainsi, en calculant l'écart entre les maxima, on peut obtenir une information aidant à évaluer l'état de vieillissement de la peau.

Bien entendu, l'invention n'est pas limitée aux exemples de réalisation qui viennent d'être donnés. On peut notamment utiliser un capteur autre que celui commercialisé sous la marque TOUCHCHIP® .

Dans toute la description, y compris les revendications, l'expression « comportant un » doit être comprise comme étant synonyme de « comportant au moins un », sauf si le contraire est spécifié.

## Revendications

1. Dispositif permettant à un utilisateur d'évaluer l'hydratation d'une région de la peau ou des muqueuses, ce dispositif comprenant un capteur (10) comportant un réseau de cellules de détection non optiques (11) et un dispositif de traitement agencé pour délivrer à cet utilisateur du dispositif au moins une information relative à l'homogénéité de l'hydratation de ladite région à partir de signaux provenant dudit capteur.

2. Dispositif selon la revendication 1, **caractérisé par le fait que** le capteur présente une résolution spatiale meilleure que 100 µm, dans au moins une direction.

3. Dispositif selon la revendication précédente, **caractérisé par le fait que** le capteur présente une résolution spatiale égale à/ou meilleure que 50 µm dans au moins une direction.

4. Dispositif selon l'une quelconque des revendications précédentes, **caractérisé par le fait que** le réseau est bidimensionnel.

5. Dispositif selon l'une quelconque des revendications précédentes, **caractérisé par le fait qu'**il est agencé pour délivrer au moins une image (60) de la région analysée par le capteur.

6. Dispositif selon l'une quelconque des revendications précédentes, **caractérisé par le fait qu'**il est agencé pour analyser l'évolution de l'hydratation au cours du temps avec le capteur en place sur la région analysée.

7. Dispositif selon la revendication 6, **caractérisé par le fait qu'**il est agencé pour déterminer à partir de l'évolution de l'hydratation au cours du temps une information relative à la tendance à transpirer de l'individu examiné.

8. Dispositif selon l'une quelconque des revendications 5 à 7, **caractérisé par le fait que** le dispositif de traitement est agencé pour effectuer un traitement statistique de ladite image et **par le fait que** ladite information résulte au moins de ce traitement statistique.

9. Dispositif selon la revendication 8, **caractérisé par le fait que** le traitement statistique comporte la détermination du niveau de gris (G) partagé par le plus grand nombre de pixels de l'image.

10. Dispositif selon l'une quelconque des revendications précédentes, **caractérisé par le fait que** lesdites cellules de détection sont capacitives.

11. Dispositif selon l'une quelconque des revendications 5 à 10, **caractérisé par le fait que** l'on détermine les niveaux de gris moyens de sous-ensembles (100), de préférence non sécants, de l'image et que l'on calcule une valeur représentative d'un écart entre ces niveaux de gris moyens, de préférence l'écart type:

12. Dispositif selon la revendication précédente, **caractérisé par le fait que** les différents sous-ensembles (100) sont formés par un quadrillage d'au moins une partie de l'image.

13. Dispositif selon la revendication 11, **caractérisé par le fait que** les sous-ensembles (100) sont disposés sur l'image d'une façon non régulière.

14. Dispositif selon l'une quelconque des revendications 5 à 13 **caractérisé par le fait qu'**il est agencé pour comparer l'image (60) avec dés images d'une banque d'images et, au moins à partir de cette comparaison, en tirer une information utile pour évaluer l'état de la peau ou des muqueuses.

15. Dispositif selon l'une quelconque des revendications précédentes, **caractérisé par le fait qu'**il est agencé pour délivrer une indication relative à l'état de vieillissement de la peau.

16. Dispositif selon la revendication précédente, **caractérisé par le fait que** le dispositif de traitement est agencé pour permettre de déterminer deux orientations prépondérantes (α₁, α₂) des lignes de la peau et l'écart existant entre lesdites orientations.

17. Dispositif selon la revendication 16, **caractérisé par le fait qu'**il est agencé pour délivrer à partir dudit écart une indication relative à l'état de vieillissement de la peau.

18. Dispositif selon l'une quelconque des revendications précédentes, **caractérisé par le fait qu'**il est agencé pour que le capteur (10) analyse ladite région pendant une durée prédéfinie.

19. Dispositif selon la revendication précédente, **caractérisé par le fait que** la durée prédéfinie est comprise entre 1 et 30 secondes, notamment entre 2 et 10 secondes, voire entre 3 et 7 secondes.

20. Dispositif selon l'une quelconque des revendications précédentes, **caractérisé par le fait que** le capteur comporte plus de 200 x 200 cellules de détection capacitives.

21. Dispositif selon l'une quelconque des revendications précédentes, **caractérisé par le fait que** le réseau de cellules de détection capacitives (11) occupe une surface comprise entre 10 x 10 mm² et 20 x 20 mm².

22. Dispositif selon l'une quelconque des revendications précédentes, **caractérisé par le fait qu'**il est agencé pour échanger des données avec un ordinateur personnel ou avec un terminal portable, notamment un téléphone portable.

23. Dispositif selon l'une quelconque des revendications précédentes, **caractérisé par le fait qu'**il comporte au moins un second capteur (19) d'un type différent, notamment un capteur de conductivité, de température, de couleur, d'élasticité, de pH ou un biocapteur.

24. Procédé pour évaluer l'hydratation d'une région non dermatoglyphique de la peau ou des muqueuses, notamment de l'avant-bras, comportant les étapes suivantes :
- appliquer sur ladite région un capteur (10) comportant un réseau de cellules de détection non optiques, de préférence capacitives (11),
- recueillir des signaux provenant dudit capteur et délivrer à partir de ces signaux une information relative à l'homogénéité de l'hydratation de ladite région sous la forme d'un message sonore et/ou visuel.

25. Procédé selon la revendication 24, **caractérisé par le fait que** l'on détermine l'information relative à l'hydratation de ladite région à partir au moins d'un traitement statistique d'au moins une image obtenue à l'aide dudit capteur (10).

26. Procédé selon l'une des revendications 24 et 25, **caractérisé par le fait que** l'on détermine à partir des signaux provenant du capteur (10) le vieillissement de la peau.

27. Procédé selon l'une quelconque des revendications 24 à 26, **caractérisé par le fait que** l'on analyse ladite région avec le capteur (10) pendant une durée prédéfinie.

28. Procédé selon l'une quelconque des revendications 24 à 27 **caractérisé par le fait que** l'on détermine à partir des signaux délivrés par le capteur les orientations prépondérantes (α₁, α₂) des lignes (D) de la peau.

29. Procédé selon la revendication précédente, **caractérisé par le fait que** l'on calcule un écart entre lesdites orientations.

30. Procédé selon l'une quelconque des revendications 24 à 29, **caractérisé par le fait que** l'on compare les résultats de deux évaluations relatives à l'hydratation de ladite région à deux instants différents et **par le fait que** l'on délivre une indication liée à l'évolution de l'hydratation entre ces deux instants.

31. Procédé selon l'une quelconque des revendications 24 à 30, **caractérisé par le fait que** l'on effectue au moins une évaluation de l'hydratation d'une région du corps non exposée au soleil et au moins une évaluation d'une région du corps exposée au soleil, et **par le fait que** l'on compare les résultats.

32. Procédé selon la revendication précédente, **caractérisé par le fait que** l'on détermine à partir de la comparaison des résultats le vieillissement de la peau.

33. Procédé selon l'une quelconque des revendications 24 à 32, **caractérisé par le fait que** l'on analyse ladite région avec le capteur en un premier emplacement géographique et **par le fait que** l'on transmet à distance par un réseau Internet, Intranet ou de téléphonie mobile, des données obtenues à partir des signaux délivrés par le capteur, et **par le fait que** l'on traite à un deuxième emplacement géographique lesdites données de manière à évaluer l'hydratation de ladite région.

34. Procédé selon l'une quelconque des revendications 24 à 33, **caractérisé par le fait que** l'on mémorise des images successives dans le temps de la région étudiée, obtenues à partir dudit capteur.

35. Procédé selon la revendication précédente, **caractérisé par le fait que** l'on compare au moins deux valeurs obtenues à partir d'un traitement statistique de deux images.

36. Procédé selon l'une quelconque des revendications 24 à 35, **caractérisé par le fait que** l'information est délivrée sous la forme d'un message sonore et/ou visuel.

37. Procédé pour déterminer l'efficacité d'un traitement ayant une action sur l'hydratation de la peau, comportant les étapes suivantes :
- effectuer une première évaluation de l'hydratation de la peau,
- effectuer le traitement,
- effectuer après le traitement, une deuxième évaluation de l'hydratation de ladite région,
- l'une au moins des première et deuxième évaluations étant effectuée en mettant en oeuvre le procédé tel que défini dans l'une quelconque des revendications 24 à 36.

38. Procédé selon la revendication précédente, **caractérisé par le fait qu'**à la fois les première et deuxième évaluations sont effectuées en mettant en oeuvre le procédé tel que défini dans l'une quelconque des revendications 24 à 36.

39. Procédé de traitement cosmétique d'une région du corps, **caractérisé par le fait qu'**il comporte les étapes suivantes :
- évaluer l'hydratation de ladite région en mettant en oeuvre le procédé tel que défini dans l'une quelconque des revendications 24 à 36.
- effectuer un traitement ayant une action sur l'hydratation de la peau au vu du résultat de l'évaluation.

40. Procédé pour évaluer l'hydratation de la peau, dans lequel on utilise un capteur d'empreintes digitales à réseau de cellules de détection capacitives, intégré dans un micro-ordinateur ou un terminal portable, notamment un téléphone portable, pour fournir une information relative à l'homogénéité de l'hydratation de la peau en mettant en oeuvre le procédé tel que défini, dans l'une quelconque des revendications 25 à 36.

41. Téléphone portable équipé d'un capteur comportant un réseau de cellules de détection capacitives destiné à être appliqué sur une région de la peau ou des muqueuses, ce téléphone comportant des moyens pour traiter des signaux délivrés par lendit capteur afin de délivrer à un utilisateur une information relative à l'homogénéité de l'hydratation de la peau ou des muqueuses.

## Claims

1. Apparatus enabling a user to evaluate the hydration of a region of the skin or the mucous membranes, the apparatus comprising a sensor (10) comprising an array of non-optical detection cells (11), and processor apparatus arranged to deliver to the user at least one piece of information relating to the uniformity of hydration of said region on the basis of signals coming from said sensor.

2. Apparatus according to claim 1, wherein the sensor presents spatial resolution better than 100 µm, in at least one direction.

3. Apparatus according to the preceding claim, wherein the sensor presents spatial resolution equal to or better than 50 µm, in at least one direction.

4. Apparatus according to any preceding claim, wherein the array is two-dimensional.

5. Apparatus according to any preceding claim, the apparatus being arranged to deliver at least one image (60) of the region analyzed by the sensor.

6. Apparatus according to any preceding claim, the apparatus being arranged to analyze variation in hydration over time with the sensor in place on the region under analysis.

7. Apparatus according to claim 6, the apparatus being arranged to determine information relating to the tendency to transpire of the individual under examination on the basis of the way in which hydration varies over time.

8. Apparatus according to any claim 5 to 7, wherein the processor apparatus is arranged to perform statistical processing of said image, and wherein said information results at least from said statistical processing.

9. Apparatus according to claim 8, wherein the statistical processing comprises determining the gray level shared by the greatest number of pixels in the image.

10. Apparatus according to any preceding claim, wherein the non-optical detection cells comprise capacitive detection cells.

11. Apparatus according to any claim 5 to 10, wherein mean gray levels of subsets of the image (100) preferably non-intersecting, are determined and a value, preferably standard deviation, is calculated that is representative of a difference between said mean gray levels.

12. Apparatus according to claim 11, wherein the intersecting subsets are formed by a rectangular grid over at least a portion of the image.

13. Apparatus according to claim 11, wherein the subsets are disposed in irregular manner over the image.

14. Apparatus according to any claim 5 to 13, the apparatus being arranged to compare the image (60) with images in a bank of images, and at least on the basis of said comparison, to derive information that is useful for evaluating the state of the skin or of the mucous membranes.

15. Apparatus according to any preceding claim, the apparatus being arranged to deliver an indication relating to the state of aging of the skin.

16. Apparatus according to the preceding claim, wherein the processing apparatus is arranged to determine two major directions of skin lines (α1, α2) and the difference that exists between said directions.

17. Apparatus according to claim 16, the apparatus being arranged to deliver an indication relating to the state of aging of the skin from said difference.

18. Apparatus according to any preceding claim, the apparatus being arranged to enable the sensor to analyze said region for a predefined duration.

19. Apparatus according to the preceding claim, wherein the predefined duration lies in the range from about 1 s to about 30 s, in particular in the range 2 s to 10 s, even in the range 3 s to 7 s.

20. Apparatus according to any preceding claim, wherein the sensor comprises more than 200 × 200 capacitive detection cells.

21. Apparatus according to any preceding claim, wherein the array of capacitive detection cells (11) occupies an area lying in the range from about 10 mm × 10 mm to about 20 mm × 20 mm.

22. Apparatus according to any preceding claim, the apparatus being arranged to exchange data with a personal computer or a portable terminal, in particular a mobile telephone.

23. Apparatus according to claim 1, including at least one second sensor (19) of a different type, in particular a biosensor or a sensor of conductivity, temperature, color, elasticity or pH.

24. A method of evaluating the hydration of a non-dermatoglyphic region of the skin or the mucous membranes in particular the forearm, the method comprising:
applying to said region a sensor (10) comprising an array of non-optical detection cells preferably capacitive detection cells (11); and
picking up signals coming from said sensor and, on the basis of said signals, delivering information relating to the uniformity of hydration of said region, via a voiced and/or visual message.

25. A method according to claim 24, wherein the information relating to hydration of said region is determined at least from statistical processing of at least one image obtained using said sensor (10).

26. A method according to any claim 24 or 25, wherein the aging of the skin is determined from the signals coming from the sensor (10).

27. A method according to any claim 24 to 26, wherein said region is analyzed with the sensor (10) for a predefined duration.

28. A method according to any claim 24 to 27, wherein the major directions (α1, α2) of the skin lines are determined from the signals delivered by the sensor.

29. A method according to the preceding claim, wherein a difference is calculated between said directions.

30. A method according to any claim 24 to 29, wherein the results of two evaluations relating to the hydration of said region at two different instants are compared, and wherein an indication associated with the variation in hydration between said two instants is delivered.

31. A method according to any claim 24 to 30, wherein at least one evaluation is performed of the hydration of a region of the body that is not exposed to the sun and at least one evaluation is performed of a region of the body that is exposed to the sun, and wherein the results are compared.

32. A method according to the preceding claim, wherein the aging of the skin is determined by comparing the results.

33. A method according to claim 32, wherein said region is analyzed with the sensor at a first geographical location, wherein the data obtained from the signals delivered by the sensor is transmitted remotely via an Internet, Intranet, or mobile telephony network and wherein said data is processed at a second geographical location in order to evaluate the hydration of said region.

34. A method according to any claim 24 to 33, wherein images obtained successively in time of the region under study by said sensor are stored.

35. A method according to the preceding claim, wherein at least two values obtained statistical processing of two images are compared.

36. A method according to any claim 24 to 35, wherein the method comprises issuing a voiced and/or visual message containing the information.

37. A method of determining the effectiveness of treatment having an action on skin hydration, the method comprising:
• performing a first evaluation of skin hydration;
• performing the treatment;
• after the treatment, performing a second evaluation of the hydration of said region; and
• at least one of the first and second evaluations being performed by implementing a method as defined in any claim 24 to 36.

38. A method according to claim 48, wherein both the first and the second evaluations are performed by implementing the method as defined in any claim 24 to 36.

39. A method of treating a region of the body, the method comprising:
• evaluating the hydration of said region by implementing the method as defined in any claim 24 to 36; and
• performing treatment that has action on the hydration of the skin in the light of the result of the evaluation.

40. A method of evaluating skin hydration, in which method a fingerprint sensor is used having an array of capacitive detection cells integrated in at least one of a computer and a portable terminal, in particular a mobile telephone for providing information relating to the hydration of the skin.

41. A mobile telephone fitted with a sensor comprising an array of capacitive detection cells for application to a region of the skin or the mucous membranes, said telephone including means for processing signals delivered by said sensor in order to deliver to a user information relating to the hydration of the skin or the mucous membranes.

## Patentansprüche

1. Vorrichtung, die es einem Anwender ermöglicht, die Hydratation bzw. Hydrierung eines Bereiches der Haut oder der Schleimhäute zu beurteilen, wobei diese Vorrichtung aufweist, einen Messfühler (10), der ein Raster von nicht optischen Messzellen (11) und eine Behandlungseinrichtung aufweist, um dem Anwender der Vorrichtung zumindest eine Information im Verhältnis zu der Homogenität der Hydratation bzw. Hydrierung in dem besagten Bereich anhand von Signalen zu liefern, die von dem Messfühler stammen.

2. Vorrichtung nach Anspruch 1, **dadurch gekennzeichnet, dass** der Messfühler eine räumliche Auflösung vorgibt, die in zumindest einer Richtung besser als 100 µm ist.

3. Vorrichtung nach dem voranstehenden Anspruch, **dadurch gekennzeichnet, dass** der Messfühler eine räumliche Auflösung vorgibt, die in zumindest einer Richtung gleich 50 µm oder besser ist.

4. Vorrichtung nach irgendeinem der voranstehenden Ansprüche, **dadurch gekennzeichnet, dass** das Raster zweidimensional ist.

5. Vorrichtung nach irgendeinem der voranstehenden Ansprüche, **dadurch gekennzeichnet, dass** diese ausgebildet ist, um zumindest ein Bild (60) des analysierten Bereiches durch den Messfühler zu liefern.

6. Vorrichtung nach irgendeinem der voranstehenden Ansprüche, **dadurch gekennzeichnet, dass** diese ausgebildet ist, um die Entwicklung der Hydratation bzw. Hydrierung im Verlaufe der Zeit mit dem in Stellung gebrachten Messfühler auf dem analysierten Bereich zu analysieren.

7. Vorrichtung nach Anspruch 6, **dadurch gekennzeichnet, dass** diese ausgebildet ist, um anhand der Entwicklung der Hydratation bzw. Hydrierung im Verlaufe der Zeit eine Information relativ zu der Tendenz zum Transpirieren des untersuchten Individuums zu bestimmen.

8. Vorrichtung nach irgendeinem der Ansprüche 5 bis 7, **dadurch gekennzeichnet, dass** die Behandlungseinrichtung ausgebildet ist, um eine statistische Behandlung des besagten Bildes zu bewirken, und **dadurch**, dass sich die besagte Information zumindest aus der statischen Behandlung bzw. Verarbeitung ergibt.

9. Vorrichtung nach Anspruch 8, **dadurch gekennzeichnet, dass** die statistische Behandlung bzw. Verarbeitung die Bestimmung des Grauniveaus (G) aufweist, dass sich die größte Anzahl der Bildelemente des Bildes teilen.

10. Vorrichtung nach irgendeinem der voranstehenden Ansprüche, **dadurch gekennzeichnet, dass** die besagten Messzellen kapazitiv sind.

11. Vorrichtung nach irgendeinem der Ansprüche 5 bis 10, **dadurch gekennzeichnet, dass** man die Grauniveaus im Mittel von den Unter- bzw. Teilmengen (100), bevorzugt den nicht geschnittenen, des Bildes bestimmt und dass man einen Wert berechnet, der repräsentativ für eine Abweichung unter den Grauniveaumittelwerten ist, bevorzugt die Standardabweichung.

12. Vorrichtung nach dem voranstehenden Anspruch, **dadurch gekennzeichnet, dass** die verschiedenen Unter- bzw. Teilmengen (100) durch eine Quadrierung von zumindest einem Teil des Bildes gebildet werden.

13. Vorrichtung nach Anspruch 11, **dadurch gekennzeichnet, dass** die Unter- bzw. Teilmengen (100) auf dem Bild in einer nicht regulären bzw. gleichmäßigen Weise angeordnet sind.

14. Vorrichtung nach irgendeinem der Ansprüche 5 bis 13, **dadurch gekennzeichnet, dass** diese ausgebildet ist, um das Bild (60) mit den Bildern einer Bilddatenbank zu vergleichen, und dass zumindest aufgrund dieses Vergleichs eine Information herbeigezogen wird, die zum Bewerten des Zustandes der Haut oder der Schleimhäute nützlich ist.

15. Vorrichtung nach irgendeinem der voranstehenden Ansprüche, **dadurch gekennzeichnet, dass** diese ausgebildet ist, um einen Hinweis im Verhältnis zu dem Zustand des Alterns der Haut zu liefern.

16. Vorrichtung nach dem voranstehenden Anspruch, **dadurch gekennzeichnet, dass** die Behandlungsvorrichtung angeordnet ist, um zu ermöglichen, zwei vorherrschende Orientierungen (α₁, α₂) der Linien der Haut und die vorkommende Abweichung zwischen den besagten Orientierungen zu bestimmen.

17. Vorrichtung nach Anspruch 16, **dadurch gekennzeichnet, dass** diese angeordnet ist, um aus der Abweichung einen Hinweis relativ zu dem Zustand der Alterung der Haut zu liefern.

18. Vorrichtung nach irgendeinem der voranstehenden Ansprüche, **dadurch gekennzeichnet, dass** diese ausgebildet ist, damit der Messfühler (10) den besagten Bereich während einer vorbestimmten Zeitdauer analysiert.

19. Vorrichtung nach dem voranstehenden Anspruch, **dadurch gekennzeichnet, dass** die vorbestimmte Zeitdauer enthalten ist zwischen 1 und 30 Sekunden, insbesondere zwischen 2 und 10 Sekunden, bevorzugt zwischen 2 und 7 Sekunden.

20. Vorrichtung nach irgendeinem der voranstehenden Ansprüche, **dadurch gekennzeichnet, dass** der Messfühler mehr als 200 x 200 kapazitive Messzellen aufweist.

21. Vorrichtung nach irgendeinem der voranstehenden Ansprüche, **dadurch gekennzeichnet, dass** das Raster der kapazitiven Messzellen (11) eine Oberfläche besetzt, die zwischen 10 x 10 mm² und 20 x 20 mm² liegt.

22. Vorrichtung nach irgendeinem der voranstehenden Ansprüche, **dadurch gekennzeichnet, dass** diese ausgebildet ist, um Daten mit einem Computer bzw. PC oder einem tragbaren Terminal, insbesondere einem tragbaren Telefon bzw. Handy auszutauschen.

23. Vorrichtung nach irgendeinem der voranstehenden Ansprüche, **dadurch gekennzeichnet, dass** diese zumindest einen zweiten Messfühler (19) von einer anderen Art aufweist, insbesondere einen leitenden Messfühler, Temperaturmessftihler, Farbmessfühler, Elastizitätsmessfühler, Messfühler für den ph-Wert oder einen Biomessfühler.

24. Verfahren zum Bewerten der Hydratation bzw. Hydrierung eines nicht dermatoglyphen bzw. mit Hautlinien versehenen Bereiches der Haut oder der Schleimhäute, insbesondere des Unterarms, das die folgenden Schritte aufweist:
- auf dem besagten Bereich wird ein Messfühler (10) angewandt, der ein Raster von nicht optischen Messzellen, bevorzugt kapazitiven Messzellen (11), aufweist,
- Aufnehmen von Signalen, die von dem Messfühler zur Verfügung gestellt werden und auf der Grundlage dieser Signale eine Information im Verhältnis zu der Homogenität der Hydratation bzw. Hydrierung des Bereiches in der Gestalt einer Geräuschnachricht und/oder visuellen Nachricht liefern.

25. Verfahren nach dem Anspruch 24, **dadurch gekennzeichnet, dass** die Information relativ zu der Hydratation bzw. Hydrierung des Bereiches auf der Grundlage von zumindest einer statistischen Behandlung bzw. Verarbeitung von zumindest einem Bild bestimmt wird, das durch die Hilfe des Messfühlers (10) erhalten wird.

26. Verfahren nach einem der Ansprüche 24 und 25, **dadurch gekennzeichnet, dass** auf der Grundlage der Signale, die von dem Messfühler (10) erhalten worden sind, die Alterung der Haut bestimmt wird.

27. Verfahren nach irgendeinem der Ansprüche 24 bis 26, **dadurch gekennzeichnet, dass** der Bereich mit dem Messfühler (10) während einer vorbestimmten Zeitdauer analysiert wird.

28. Verfahren nach irgendeinem der Ansprüche 24 bis 27, **dadurch gekennzeichnet, dass** auf der Grundlage der von dem Messfühler gelieferten Signale die vorherrschenden Orientierungen (α₁, α₂) der Linien (D) der Haut bestimmt werden.

29. Verfahren nach dem voranstehenden Anspruch, **dadurch gekennzeichnet, dass** eine Abweichung zwischen den Orientierungen berechnet wird.

30. Verfahren nach irgendeinem der Ansprüche 24 bis 29, **dadurch gekennzeichnet, dass** die Ergebnisse der Auswertungen im Verhältnis zur Hydratation bzw. Hydrierung des Bereiches zu zwei verschiedenen Zeitpunkten verglichen werden, und **dadurch**, dass ein Hinweis geliefert wird, der in der Entwicklung der Hydratation bzw. Hydrierung zwischen den zwei Zeitpunkten liegt.

31. Verfahren nach irgendeinem der Ansprüche 24 bis 30, **dadurch gekennzeichnet, dass** zumindest eine Bewertung der Hydratation bzw. Hydrierung eines Bereiches des Körpers, der der Sonne nicht ausgesetzt ist und zumindest eine Bewertung eines Bereiches des Körpers bewirkt wird, der der Sonne ausgesetzt ist, und **dadurch**, dass die Ergebnisse verglichen werden.

32. Verfahren nach dem voranstehenden Anspruch, **dadurch gekennzeichnet, dass** aufgrund des Vergleiches der Ergebnisse die Alterung der Haut bestimmt wird.

33. Verfahren nach irgendeinem der Ansprüche 24 bis 32, **dadurch gekennzeichnet, dass** die Analyse des Bereiches mit dem Messfühler an einem ersten geographischen Ort durchgeführt wird und **dadurch**, dass über eine Distanz über ein Intemet-, Intranet- oder Mobiltelefonnetzwerk die Daten übertragen werden, die durch die Signale erhalten worden sind, die durch den Messfühler geliefert worden sind, und **dadurch**, dass an einem zweiten geographischen Ort die Daten in der Weise behandelt werden, um die Hydratation bzw. Hydrierung des Bereiches zu bewerten.

34. Verfahren nach irgendeinem der Ansprüche 24 bis 33, **dadurch gekennzeichnet, dass** man die zeitlich aufeinander folgenden Bilder des geprüften Bereiches speichert, die durch den Messfühler erhalten worden sind.

35. Verfahren nach dem voranstehenden Anspruch, **dadurch gekennzeichnet, dass** zumindest zwei durch eine statistische Behandlung bzw. Auswertung der zwei Bilder erhaltene Werte verglichen werden.

36. Verfahren nach irgendeinem der Ansprüche 24 bis 34, **dadurch gekennzeichnet, dass** die Information in der Gestalt einer Geräuschnachricht und/oder einer visuellen Nachricht geliefert wird.

37. Verfahren zur Bestimmung der Wirksamkeit einer Behandlung, die eine Wirkung auf die Hydratation bzw. Hydrierung der Haut hat, das die nachfolgenden Schritte aufweist:
- eine erste Bewertung der Hydratation bzw. Hydrierung der Haut wird bewirkt,
- die Behandlung wird bewirkt,
- nach der Behandlung wird eine zweite Bewertung der Hydratation bzw. Hydrierung des Bereiches bewirkt,
- zumindest eine der ersten und zweiten Bewertungen wird durchgerührt, indem das Verfahren in die Tat umgesetzt wird, wie es in einem der Ansprüche 24 bis 36 definiert ist.

38. Verfahren nach dem voranstehenden Anspruch, **dadurch gekennzeichnet, dass** bei dem Mal bei dem die erste und zweite Auswertung bewirkt werden, das Verfahren in die Tat umgesetzt wird, wie es in irgendeinem der Ansprüche 24 bis 36 definiert ist.

39. Verfahren zur kosmetischen Behandlung eines Bereichs des Körpers, **dadurch gekennzeichnet, dass** dieses die nachfolgenden Schritte aufweist:
- Bewerten der Hydratation bzw. Hydrierung des Bereiches, indem das Verfahren in die Tat umgesetzt wird, gemäß irgendeinem der Ansprüche 24 bis 36,
- Bewirken einer Behandlung, die eine Wirkung auf die Hydratation bzw. Hydrierung der Haut im Hinblick auf das Ergebnis der Bewertung hat.

40. Verfahren zur Bewertung der Hydratation bzw. Hydrierung der Haut, bei welchem ein Messfühler für Fingerabdrücke mit einen Raster von kapazitiven Messzellen verwendet wird, der in einem Mikrocomputer oder ein tragbares Terminal, insbesondere ein tragbares Telefon, integriert ist, um eine Information relativ zu der Homogenität der Hydratation bzw. Hydrierung der Haut zu liefern, indem das Verfahren in die Tat umgesetzt wird, wie es in irgendeinem der Ansprüche 25 bis 36 definiert ist.

41. Tragbares Telefon, das mit einem Messfühler ausgestattet ist, das ein Raster von kapazitiven Messzellen aufweist, das bestimmt ist, um auf einem Bereich der Haut oder der Schleimhäute eingesetzt zu werden, wobei das Telefon Mittel aufweist, um ein Signal zu verarbeiten, das durch den Messfühler geliefert worden ist, um einem Anwender eine Information im Verhältnis zu der Homogenität der Hydratation bzw. Hydrierung der Haut oder der Schleimhäute zu liefern.
